# EUROPÄISCHE PATENTANMELDUNG

(11) **EP 4 169 549 A1**
(43) Veröffentlichungstag der Anmeldung: **26.04.2023**
(21) Anmeldenummer: 22201669.3
(22) Anmeldetag: 14.10.2022
(51) Int. Cl.: A61M 5/142

(54) **DRAHTLOSES KOMMUNIKATIONSMODUL FÜR MEDIZINISCHE FLUIDPUMPE**

(30) Priorität: 19.10.2021 DE 102021127113
(71) Anmelder: B. Braun Melsungen AG, 34212 Melsungen (DE)
(72) Erfinder: SCHWARZ, Jan, 34212 Melsungen (DE); ERLEN, Christoph, 34132 Kassel (DE); NIEMEIER, Carsten, 34130 Kassel (DE); STICH, Marcel, 34329 Nieste (DE); KAUBA, Michael, 34277 Fuldabrück (DE); OSTERMOELLER, Michael, 36251 Bad Hersfeld (DE)
(74) Vertreter: Winter, Brandl - Partnerschaft mbB

(57) **Zusammenfassung**

Die medizinische Fluidpumpe (2), vorzugsweise in Form einer Infusionspumpe oder Spritzenpumpe, weist eine Platine (8), welche mit den wesentlichen Steuervorrichtungen der medizinischen Fluidpumpe (2), nämlich zumindest mit der Steuerungsvorrichtung eines Motors der medizinischen Fluidpumpe (2), bestückt ist, und einem drahtlosen Kommunikationsmodul (10) in Form einer Kommunikationsplatine (10) auf. Die Kommunikationsplatine (10) ist im Wesentlichen senkrecht bzw. orthogonal zu der Platine (8) angeordnet.

## Beschreibung

### Technisches Gebiet

Die vorliegende Offenbarung betrifft eine medizinische Fluidpumpe, insbesondere in Form einer Infusionspumpe oder Spritzenpumpe, mit einem drahtlosen Kommunikationsmodul in Form einer Kommunikationsplatine.

### Hintergrund der Erfindung

In der Medizin werden verbreitet Fluidpumpen, insbesondere Spritzenpumpen und Infusionspumpen bzw. Schlauchpumpen zur Versorgung eines Patienten mit einer definierten Dosis an Medikamenten eingesetzt.

Solche medizinischen Fluidpumpen wurden im Laufe der letzten Jahre mit immer mehr Smartfunktionen ausgerüstet. Diese Smartfunktionen werden dabei mittels zusätzlicher Platinen oder Chips implementiert, die zumeist als Zukaufteile ausgebildet sind. Diese zusätzlichen Platinen oder Chips sind vorgesehen und ausgebildet, flächig auf einer Hautplatine bzw. einem Mainboard angebracht zu werden. Daraus resultiert, dass die Hauptplatine größer ausgebildet sein muss als in dem Fall, in dem keine Smartfunktion in der medizinischen Fluidpumpe ausgebildet sind. Eine derart vergrößerte Hauptplatine benötigt eine größere Grundfläche, was dazu führt, dass die medizinische Fluidpumpe sich in ihren Abmessungen vergrößert und damit mehr Platz in Anspruch nimmt. Hierdurch ist die Zugänglichkeit eines Patienten, der mit der medizinischen Fluidpumpe versorgt wird, für medizinisches Personal verschlechtert.

Um dieses Problem zu umgehen, wurde angedacht, die Smartfunktionen direkt auf der Hauptplatine zu implementieren. Hierdurch könnte die Vergrößerung des Platzbedarfs reduziert werden. Jedoch wird dadurch die Hauptplatine erheblich verteuert und bei einem Ausfall einzelner Smartfunktionen ist direkt ein Austausch der teuren Hauptplatine notwendig.

Weiterhin wurde diskutiert, die Anzahl an Smartfunktionen zu begrenzen, um der Bauraumvergrößerung entgegenzuwirken. Dies hätte jedoch einen Verzicht auf diverse Smartfunktionen zur Folge.

### Kurzbeschreibung der Erfindung

Aufgabe und Ziel der vorliegenden Erfindung ist es demnach, eine medizinische Fluidpumpe bereitzustellen, die mit Smartfunktionen ausgerüstet ist, ohne dabei mehr Platz in Anspruch zu nehmen und ohne die oben diskutierten Nachteile aufzuweisen.

Die Aufgaben und Ziele werden hinsichtlich einer gattungsgemäßen medizinischen Fluidpumpe offenbarungsgemäß durch den Gegenstand des Anspruchs 1 gelöst.

Die medizinische Fluidpumpe, vorzugsweise in Form einer Infusionspumpe oder Spritzenpumpe, beinhaltet eine Platine, welche mit den wesentlichen Steuerungsvorrichtungen der medizinischen Fluidpumpe, nämlich zumindest mit der Steuerungsvorrichtung eines Motors der medizinischen Fluidpumpe, bestückt ist, und ein drahtloses Kommunikationsmodul in Form einer Kommunikationsplatine. Die Kommunikationsplatine ist im Wesentlichen senkrecht bzw. orthogonal zu der Platine angeordnet.

In anderen Worten weist die medizinische Fluidpumpe eine Platine auf, welche auch als Mainboard oder Hauptplatine bezeichnet werden kann. Auf dieser Platine sind die für die Funktion der medizinischen Fluidpumpe essenziellen Steuerungsvorrichtungen implementiert. Unter die essenziellen Steuerungsvorrichtungen für die Funktion der medizinischen Fluidpumpe fällt zumindest die Steuerungsvorrichtung des Motors der Fluidpumpe, welcher für die Applikation des Fluids bzw. des Medikaments verantwortlich ist. Vorzugsweise kann neben der Steuerungsvorrichtung für den Motor der Fluidpumpe auch eine Überwachung des Motors auf der Fluidpumpe auf der Platine realisiert sein. Zusätzlich beinhaltet die medizinische Fluidpumpe in einer Menge von Smartfunktionen implementierenden Smartplatinen das drahtlose Kommunikationsmodul in Form der Kommunikationsplatine. Die Kommunikationsplatine beinhaltet verschiedene Vorrichtungen, die eine drahtlose Kommunikation von Daten vorzugsweise mittels WLAN und / oder Bluetooth und / oder einer beliebigen drahtlosen Funktechnologie zwischen der medizinischen Fluidpumpe und einer externen Vorrichtung ermöglicht. Die Kommunikationsplatine ist in ihrer flächigen Ausdehnung im Wesentlichen senkrecht bzw. orthogonal zu der flächigen Ausdehnung der Platine angeordnet. In nochmals anderen Worten beträgt der Winkel zwischen der flächigen Ausdehnung der Kommunikationsplatine und der flächigen Ausdehnung der Platine zwischen 80° und 100°, vorzugsweise zwischen 85° und 95° und insbesondere vorzugsweise 90°.

Es hat sich für die Anmelderin gezeigt, dass insbesondere die Kommunikationsplatine eine der für die Smartfunktionen notwendigen Smartplatinen ist, die einen erheblichen Platzbedarf aufweist. Durch eine Auswahl der Kommunikationsplatine aus einer Menge der Smartfunktionen umsetzenden Platinen und die senkrechte bzw. orthogonale Anordnung der Kommunikationsplatine zu der Platine ist die auf die Platine projizierte Grundfläche der Kommunikationsplatine erheblich reduziert und die Platine kann kompakter gestaltet werden als in dem Fall, in dem die Kommunikationsplatine flächig / parallel zu der Platine ausgerichtet ist. Hierdurch kann gewährleistet werden, dass sich die Abmessungen der medizinischen Fluidpumpe nicht ändern zu dem Fall, in dem kein drahtloses Kommunikationsmodul in der medizinischen Fluidpumpe vorgesehen ist. Ferner kann durch die orthogonale Ausrichtung der Kommunikationsplatine zu der Platine Luft besser um die Kommunikationsplatine zirkulieren als in dem Fall der flächigen / parallelen Ausrichtung. Hierdurch ist die Kühlung der Kommunikationsplatine verbessert. Eine solche Ausrichtung der Kommunikationsplatine steht in einem direkten Gegensatz zu der sonst üblichen Ausrichtung der Kommunikationsplatine.

In einem ersten Aspekt kann die Kommunikationsplatine von einem metallischen oder metallische Elemente beinhaltenden (Schirm-)Käfig umschlossen bzw. umgeben sein.

In anderen Worten ist die Kommunikationsplatine von metallischen Blechen oder einem metallischen Gitterkäfig oder einem Kunststoffkäfig, welcher metallische Elemente, beispielsweise in Form von Metallpartikel, enthält, umschlossen.

In nochmals anderen Worten ist die Kommunikationsplatine insbesondere gegen hochfrequente elektromagnetische Felder mit Hilfe von metallischen Elementen abgeschirmt. Zusätzlich wird auch von der Kommunikationsplatine ausgehende Störausstrahlung abgeschirmt. Durch die Abschirmung der Kommunikationsplatine gegen elektromagnetische Strahlung / Felder kann gewährleistet werden, dass die Funktion der Kommunikationsplatine nicht durch elektromagnetische Strahlung aus der Umgebung beeinträchtigt wird. Anders ausgedrückt sorgt der Käfig für eine optimierte EMV / ESD-Stabilität.

In einem weiteren Aspekt kann der Käfig an einzelnen voneinander beabstandeten Punkten, insbesondere an vier Punkten, mit der Kommunikationsplatine verbunden sein.

In anderen Worten ist der Käfig an vier Anbindungspunkten, die sich vorzugsweise in den vier Ecken der Kommunikationsplatine befinden, mit der Kommunikationsplatine verbunden.

In einem weiteren Aspekt kann der Käfig an zwei Punkten stoffschlüssig, insbesondere durch Löten, mit der Kommunikationsplatine verbunden sein und an zwei Punkten kraftschlüssig, insbesondere durch Klemmen, mit der Kommunikationsplatine verbunden sein.

In anderen Worten ist der Käfig an zwei sich an derselben Kante der Kommunikationsplatine befindlichen Anbindungspunkten stoffschlüssig mit der Kommunikationsplatine verbunden. An zwei weiteren Anbindungspunkten der Kommunikationsplatine, die sich an einer weiteren Kante der Kommunikationsplatine befinden, ist der Käfig kraftschlüssig mit der Kommunikationsplatine verbunden.

Die Verbindung mittels Kraftschluss ermöglich bei zwischen Käfig und Kommunikationsplatine auftretenden Kräften eine Verschiebung der kraftschlüssig verbundenen Verbindungspunkte, wenn die auftretende Kraft einen gewissen Schwellenwert übersteigt. Auf diese Weise ist gewährleistet, dass bei einer möglichen Ausdehnung des metallischen Käfigs, beispielsweise auf Grund von einer Temperaturerhöhung, keine übermäßig großen Spannungen in die Kommunikationsplatine eingeleitet werden, welche die Kommunikationsplatine beschädigen könnten.

In einem weiteren Aspekt kann der Käfig mit einem Massepotential der Platine verbunden sein.

In anderen Worten ist der Käfig elektrisch leitend mit der Masse bzw. dem Ground (GND) der Platine verbunden.

In einem weiteren Aspekt kann die Kommunikationsplatine mit mehreren, insbesondere zwei, Antennen verbunden sein.

In anderen Worten ist mehr als eine Antenne vorgesehen, die mit der Kommunikationsplatine verbunden ist. Bevorzugt handelt es sich um zwei Antennen, wobei die Antennen getrennt von der Kommunikationsplatine ausgebildet sind.

In nochmals anderen Worten verfügt die Kommunikationsplatine über MIMO (Multiple Input Multiple Output) Schnittstellen. Dies ermöglicht Codierungsverfahren, die neben einer zeitlichen auch eine räumliche Dimension zur Informationsübertragung nutzen. Hierdurch lässt sich sowohl die Qualität in Form der Bitfehlerhäufigkeit als auch die Datenrate der drahtlosen Verbindung erheblich verbessern.

Durch das getrennte Vorsehen von Antenne und Kommunikationsplatine ist es ferner möglich, die Antennen an für die Übertragungsleistung geeigneten Positionen anzuordnen.

In einen weiteren Aspekt können die mehreren, insbesondere zwei, Antennen orthogonal zueinander angeordnet sein.

In anderen Worten unterscheidet sich die räumliche Orientierung der Antennen zueinander um im Wesentlichen 90°. Durch die orthogonale Richtcharakteristik der Antennen kann die Übertragungsleistung erhöht werden. Insbesondere kann durch die Abstrahlcharakteristik der Antennen durch die orthogonale Anordnung ein Abdeckungsbereich vergrößert werden.

In einem weiteren Aspekt können die mehreren, insbesondere zwei, Antennen mittels Kabel mit der Kommunikationsplatine verbunden sein, wobei die Kabellängen der Kabel, welche die Kommunikationsplatine mit den Antennen verbinden, identisch sind.

Anders ausgedrückt werden für das Verbinden der Antennen mit der Kommunikationsplatine baugleiche Kabel eingesetzt. Die Kabellänge beträgt hierbei bevorzugt 175 mm. Dies ermöglicht einerseits eine Reduzierung der Ersatzteile und vereinfacht eine Montage der medizinischen Fluidpumpe. Andererseits ist durch die identische Kabellänge sichergestellt, dass es zu keinen Laufzeitdifferenzen zwischen den Antennen und der Kommunikationsplatine kommt. Die Kabel sind mittels Klipsen auf der Platine verlegt. Über die Klipse sind die Kabel und damit die Antennen mit dem Massepotential (Ground) der Platine verbunden.

In einem weiteren Aspekt können die Antennen in einem Abstand zueinander angeordnet sein, der einem Vielfachen einer viertel, insbesondere einem Vielfachen einer halben, Wellenlänge der Übertragungsfrequenz der Antenne entspricht.

In anderen Worten beträgt die Übertragungsfrequenz der Antennen 2,4 GHz und / oder 5 GHz. Die Wellenlänge beträgt für 2,4 GHz 12,5 cm und für 5 GHz 6 cm. Der Abstand zwischen den Antennen beträgt demnach ein Vielfaches von 3,125 cm bzw. ein Vielfaches von 3 cm. Durch eine solche Anordnung der Antennen zueinander werden gegenseitige Störeinflüsse der Antennen aufeinander minimiert.

In einem weiteren Aspekt ist die Kommunikationsplatine thermisch mit der Platine gekoppelt.

In anderen Worten ist die Kommunikationsplatine mittels eines wärmeleitfähigen Bauteils mit der Platine verbunden. Durch den hohen Metallanteil der Platine und die große Oberfläche der Platine fungiert die Platine als Kühlkörper für die Kommunikationsplatine und verhindert ein Überhitzen der Kommunikationsplatine.

In einem weiteren Aspekt kann die Kommunikationsplatine unabhängig von der Platine arbeiten.

In anderen Worten tauschen die Platine und die Kommunikationsplatine Datenpakete aus, wobei sowohl die Platine als auch die Kommunikationsplatine unabhängig voneinander funktionieren. Hierdurch ist sichergestellt, dass bei einem Ausfall oder einer temporären Störung der Kommunikationsplatine die wesentlichen Funktionen der medizinischen Fluidpumpe, die durch die Platine gesteuert werden, weiterhin ausgeführt werden können.

In einem weiteren Aspekt können die wesentlichen Funktionen der medizinischen Fluidpumpe drahtlos über die Kommunikationsplatine gesteuert werden. Insbesondere kann eine Medikamentenapplikation gestartet, pausiert oder gestoppt werden.

In einem weiteren Aspekt kann die Kommunikationsplatine Informationen über Störungen in der Funktion der Platine drahtlos beispielsweise an einen Behandler und / oder den Hersteller der Fluidpumpe übermitteln.

In einem weiteren Aspekt kann über die Kommunikationsplatine ein Software-Update für die medizinische Fluidpumpe und / oder die Medikamentendatenbank, welche auf der medizinischen Fluidpumpe hinterlegt ist, drahtlos auf die medizinische Fluidpumpe übertragen werden.

### Kurzbeschreibung der Figuren

Fig. 1 ist eine Innenansicht einer erfindungsgemäßen medizinischen Fluidpumpe.
Fig. 2 ist eine vergrößerte Darstellung eines Ausschnitts der Innenansicht der erfindungsgemäßen medizinischen Fluidpumpe mit einem drahtlosen Kommunikationsmodul in Form einer Kommunikationsplatine.
Fig. 3 ist eine weitere vergrößerte Darstellung eines Ausschnitts der Innenansicht der erfindungsgemäßen medizinischen Fluidpumpe mit dem drahtlosen Kommunikationsmodul in Form der Kommunikationsplatine und einer ersten Antenne.
Fig. 4 ist eine weitere vergrößerte Darstellung eines Ausschnitts der Innenansicht der erfindungsgemäßen medizinischen Fluidpumpe mit einer zweiten Antenne.

### Detaillierte Figurenbeschreibung

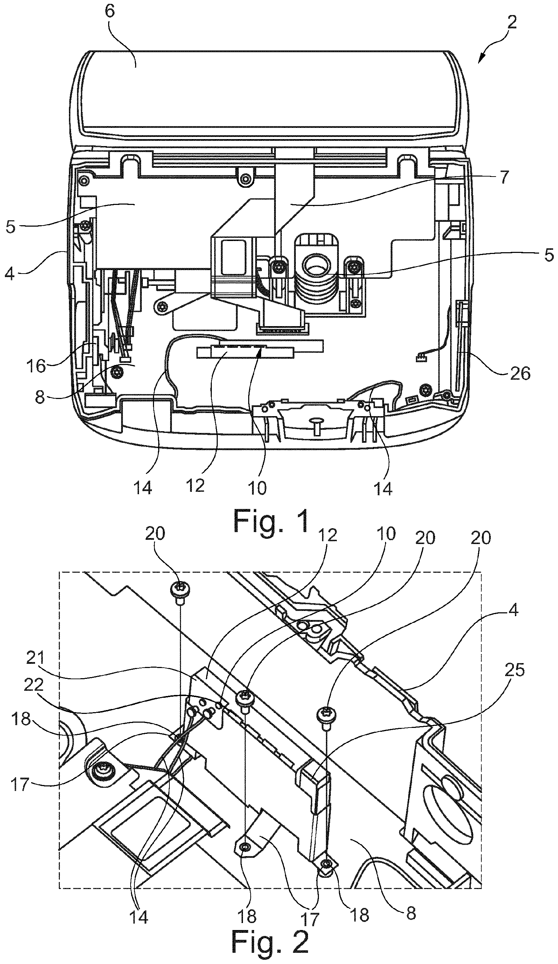

Fig. 1 zeigt eine Innenansicht einer erfindungsgemäßen medizinischen Fluidpumpe 2 mit einem im Wesentlichen rechteckigen Gehäuse 4, Antriebskomponenten 5 und einer an einer Vorderseite des Gehäuses 4 der medizinischen Fluidpumpe 2 angebrachten Frontklappe 6, welche über ein Verbindungskabel 7 verbunden ist. Die medizinische Fluidpumpe 2 beinhaltet in dem Gehäuse 4 eine Platine / Mainboard 8, welche mit den wesentlichen Steuervorrichtungen der medizinischen Fluidpumpe 2, nämlich zumindest mit der Steuerungsvorrichtung eines Motors der medizinischen Fluidpumpe 2, bestückt ist, und ein drahtloses Kommunikationsmodul in Form einer Kommunikationsplatine 10. Die Kommunikationsplatine 10 ist im Wesentlichen senkrecht bzw. orthogonal zu der Platine 8 angeordnet. Anders ausgedrückt kontaktiert eine schmale Seite der Kommunikationsplatine 10 die Fläche der Platine 8. Die Kommunikationsplatine 10 ist im Wesentlichen mittig, bezogen auf eine Breitenrichtung des Gehäuses 4, angeordnet. Die Kommunikationsplatine 10 ist von einem metallischen Schirmkäfig 12 umschlossen. Die Kommunikationsplatine 10 ist mittels Kabel 14 mit Antennen 16, 26 verbunden. Die Antennen 16, 26 sind jeweils an Seitenwänden des Gehäuses 4 der medizinischen Fluidpumpe 2 angeordnet. Ein Abstand der Antennen 16, 26 zueinander beträgt dabei ein Vielfaches einer viertel bzw. einer halben Wellenlänge der Übertragungsfrequenz der Antennen 16, 26.

Fig. 2 zeigt die Kommunikationsplatine 10 in dem Schirmkäfig 12 auf der Platine 8. Der Schirmkäfig 12 umschließt die Kommunikationsplatine 10 von sechs Seiten und verhindert, dass elektromagnetische Felder oder Strahlung die Funktion der Kommunikationsplatine 10 beeinflussen bzw. stören. Der Schirmkäfig 12 besteht aus Metallblech. Der Schirmkäfig 12 weist drei Füße 17 auf, die mit jeweils einem Durchgangsloch 18 versehen sind. Der Schirmkäfig 12 ist durch die Durchgangslöcher 18 mittels Schrauben 20 mit der Platine 8 verbunden. Durch das Vorsehen von drei Füßen 17 ist der Schirmkäfig 12 statisch bestimmt fixiert. Der Schirmkäfig 12 weist an der Platine 8 zugewandten Seite eine erste Anschlussöffnung (nicht dargestellt) auf. Durch die erste Anschlussöffnung ist die Kommunikationsplatine 10 mittels eines Platinensteckers mit der Platine 8 verbunden. Ferner weist der Schirmkäfig 12 eine zweite Anschlussöffnung 21 in einer orthogonal zu der Platine 8 orientierten Seitenfläche des Schirmkäfigs 12 auf. Durch die zweite Anschlussöffnung 21 sind die Kabel 14, welche die Kommunikationsplatine 10 mit den Antennen 16 verbinden, an der Kommunikationsplatine 10 angeschlossen. Der Schirmkäfig 12 weist ferner Ventilationsschlitze 22 auf, die einen Luftaustausch zwischen einem Inneren des Schirmkäfigs 12 und einem Innenraum des Gehäuses 4 der medizinischen Fluidpumpe verbessern, sodass ein Aufstauen von warmer Luft in dem Schirmkäfig 12 verhindert wird. In zwei in einem montierten Zustand von der Platine 8 abgewandten Eckabschnitten des Schirmkäfigs 12 sind Klemmelemente 25 ausgebildet, welche den Schirmkäfig 12 kraftschlüssig mit der Kommunikationsplatine 10 verbinden. In zwei in einem montierten Zustand der Platine 8 zugewandten Eckabschnitten des Schirmkäfigs 12 ist der Schirmkäfig 12 stoffschlüssig mit der Kommunikationsplatine 10 verbunden.

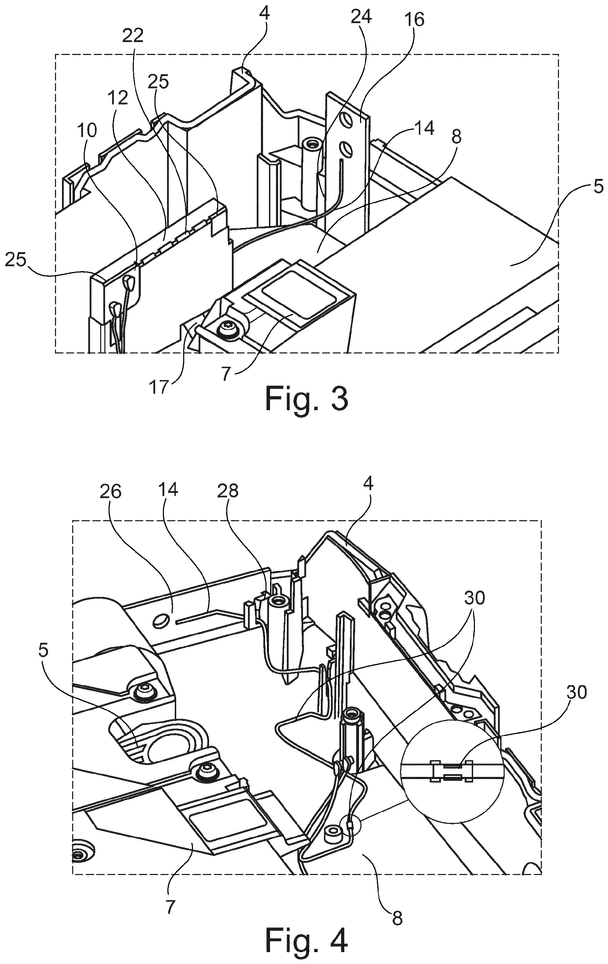

Fig. 3 zeigt die Kommunikationsplatine 10 in dem Schirmkäfig 12 auf der Platine 8 sowie eine erste mittels Kabel 14 verbundene Antenne 16. Die erste Antenne 16 ist orthogonal zu der Platine 8 ausgerichtet und an einer Innenseite der Seitenwand des Gehäuses 4 der medizinischen Fluidpumpe 2 angebracht. In dem Gehäuse 4 ist zur Aufnahme der ersten Antenne 16 eine vertikale Antennenaufnahme 24 ausgebildet, welche die Antenne 16 formschlüssig aufnimmt. Die vertikale Antennenaufnahme 24 wird von zwei Schienen gebildet, die sich in einer Richtung orthogonal zu der Platine 8 erstrecken. Die Schienen ist stoffeinstückig mit dem Gehäuse 4 ausgebildet.

Fig. 4 zeigt einen weiteren Ausschnitt des Inneren des Gehäuses 4 der medizinischen Fluidpumpe mit der Platine 8, den Kabeln 14 und einer zweiten Antenne 26, wobei die Kommunikationsplatine 10 zur besseren Übersichtlichkeit weggelassen wurde. Die zweite Antenne 26 ist parallel zu der Platine 8 ausgerichtet und damit um 90° versetzt zu der ersten Antenne 16 orientiert. Die zweite Antenne 26 ist an einer Innenseite der Seitenwand des Gehäuses 4 der medizinischen Fluidpumpe angebracht, die der ersten Antenne gegenüber liegt. In dem Gehäuse 4 ist zur Aufnahme der zweiten Antenne 26 eine horizontale Antennenaufnahme 28 ausgebildet, welche die Antenne 26 formschlüssig aufnimmt. Die horizontale Antennenaufnahme 28 wird von zwei Schienen gebildet, die sich in einer Richtung orthogonal zu der Platine 8 erstrecken. Die Schienen ist stoffeinstückig mit dem Gehäuse 4 ausgebildet. Sowohl die erste Antenne 16 als auch die zweite Antenne 26 sind mittels der Kabel 14 mit der Kommunikationsplatine 10 verbunden. Hierbei weisen das Kabel 14 zwischen der Kommunikationsplatine 10 und der ersten Antenne 16 und das Kabel 14 zwischen der Kommunikationsplatine und der zweiten Antenne 26 identische Lägen auf. Die Kabel 14 sind mittels Kabelklipsen 30 auf der Platine 8 fixiert. Mittels der Kabelklipse 30 sind die Antennen 16, 26 mit einem Massepotential der Platine 8 verbunden.

### Bezugszeichenliste

- 2: medizinische Fluidpumpe
- 4: Gehäuse
- 6: Frontklappe
- 8: Platine
- 10: Kommunikationsplatine
- 12: (Schirm-)Käfig
- 14: Kabel
- 16: (erste) Antenne
- 17: Fuß
- 18: Durchgangsloch
- 20: Schraube
- 21: Anschlussöffnung
- 22: Ventilationsschlitze
- 24: vertikale Antennenaufnahme
- 25: Klemmelement
- 26: (zweite) Antenne
- 28: horizontale Antennenaufnahme
- 30: Kabelklips

## Patentansprüche

1. Medizinische Fluidpumpe (2), vorzugsweise in Form einer Infusionspumpe oder Spritzenpumpe, mit einer Platine (8), welche mit den wesentlichen Steuervorrichtungen der medizinischen Fluidpumpe (2), nämlich zumindest mit der Steuerungsvorrichtung eines Motors der medizinischen Fluidpumpe (2), bestückt ist, und einem drahtlosen Kommunikationsmodul in Form einer Kommunikationsplatine (10), **dadurch gekennzeichnet, dass** die Kommunikationsplatine (10) im Wesentlichen senkrecht zu der Platine (8) angeordnet ist.

2. Medizinische Fluidpumpe (2) nach Anspruch 1, **dadurch gekennzeichnet, dass** die Kommunikationsplatine (10) von einem metallischen oder metallische Elemente enthaltenden Käfig (12) umschlossen ist.

3. Medizinische Fluidpumpe (2) nach Anspruch 2, **dadurch gekennzeichnet, dass** der Käfig (12) an einzelnen voneinander beabstandeten Punkten, insbesondere an vier Punkten, mit der Kommunikationsplatine (10) verbunden ist.

4. Medizinische Fluidpumpe (2) nach Anspruch 3, **dadurch gekennzeichnet, dass** der Käfig (12) an zwei Punkten stoffschlüssig, insbesondere durch Löten, mit der Kommunikationsplatine (10) verbunden ist und an zwei Punkten kraftschlüssig, insbesondere durch Klemmen, mit der Kommunikationsplatine (10) verbunden ist.

5. Medizinische Fluidpumpe (2) nach Anspruch 2 oder 3, **dadurch gekennzeichnet, dass** der Käfig (12) mit einem Massepotential der Platine (8) verbunden ist.

6. Medizinische Fluidpumpe (2) nach einem der Ansprüche 1 bis 5, **dadurch gekennzeichnet, dass** die Kommunikationsplatine (10) mit mehreren, insbesondere zwei, Antennen (16, 26) verbunden ist.

7. Medizinische Fluidpumpe (2) nach Anspruch 6, **dadurch gekennzeichnet, dass** die mehreren, insbesondere zwei, Antennen (16, 26) orthogonal zueinander angeordnet sind.

8. Medizinische Fluidpumpe (2) nach Anspruch 6 oder 7, **dadurch gekennzeichnet, dass** die mehreren, insbesondere zwei, Antennen (16, 26) mittels Kabel (14) mit der Kommunikationsplatine (10) verbunden sind, wobei die Kabellängen der Kabel (14), welche die Kommunikationsplatine (10) mit den Antennen (16, 26) verbinden, identisch sind.

9. Medizinische Fluidpumpe (2) nach einem der Ansprüche 6 bis 8, **dadurch gekennzeichnet, dass** die Antennen (16, 26) in einem Abstand zueinander angeordnet sind, der einem Vielfachen einer viertel, insbesondere einer halben, Wellenlänge der Sendefrequenz der Antennen (16, 26) entspricht.

10. Medizinische Fluidpumpe (2) nach einem der Ansprüche 1 bis 9, **dadurch gekennzeichnet, dass** die Kommunikationsplatine (10) thermisch mit der Platine (8) gekoppelt ist.
